(19) Europäisches Patentamt
European Patent Office
Office européen des brevets

(11) Publication number: **0 282 618 A1**

# (12) EUROPEAN PATENT APPLICATION

(21) Application number: **87103995.4**

(51) Int. Cl.4: **A61K 31/445**

(22) Date of filing: **18.03.87**

(43) Date of publication of application:
**21.09.88 Bulletin 88/38**

(84) Designated Contracting States:
**CH LI NL**

(71) Applicant: **Vereniging Het Nederlands Kanker Instituut**
**Plesmanlaan 121**
**NI-1066 CX Amsterdam(NL)**

Applicant: **STICHTING VRIENDEN VAN DE STICHTING DR. KARL LANDSTEINER**
**Plesmanlaan 125**
**NL-1066 CX Amsterdam(NL)**

(72) Inventor: **Miedema, Frank, Dr.**
**18622 Granvoorvisch**
**Hoofddorp(NL)**
Inventor: **Ploegh, Hidde Lolke, Dr.**
**53 Dirk van Hasselsteeg**
**Amsterdam(NL)**

(74) Representative: **Smulders, Theodorus A.H.J. et al**
**Vereenigde Octrooibureaux Nieuwe Parklaan 107**
**NL-2587 BP 's-Gravenhage(NL)**

## (54) 1-Deoxynojirimycin as an anti-HIV therapeutic.

(57) There is described the use of 1-deoxynojirimycin of the formula

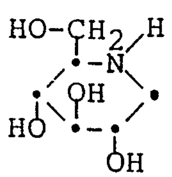

I

and pharmaceutically acceptable acid addition salts thereof, optionally, in combination with an agent that specifically interferes with the replication and/or expression of the viral genome of HIV or parts thereof for the treatment and/or prevention of AIDS and AIDS related diseases caused by HIV.

EP 0 282 618 A1

# 1-Deoxynojirimycin as an anti-HIV therapeutic

The present invention relates to the use of 1-deoxynojirimycin of the formula

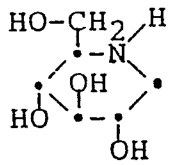

I

and pharmaceutically acceptable acid addition salts thereof, optionally, in combination with an agent that specifically interferes with the replication and/or expression of the viral genome of HIV or parts thereof for the treatment and/or prevention of AIDS and AIDS related diseases caused by HIV.

It is now well estabilished that the human immune deviciency virus (HIV) is the aetiological agent of acquired immunodeficiency syndrome (AIDS) and related diseases. HIV infects a variety of human CD4-expressing cells including $CD4^{+}$ T--cells, B-cells and monocytes. Virus infection of CD4-expresssing neural tissue has been inferred from virus isolation and in situ hybridization experiments. HIV is cytopathic for leukocytes that express relatively large amounts of CD4, but not for monocytic cells and cell lines or B-cell lines which express very little CD4. HIV-induced cytopathic effects, characterized by multinucleated giant cell formation, can be specifically inhibited by CD4 antibodies and anti-GP120 antibodies (Science 232, 1123-1127 (1986) and Nature 323, 725-728 (1986)). It has been shown that uninfected $CD4^{+}$ cells can be incorporated in syncytia by GP120-and CD4-dependent fusion with HIV-infected cells. For envelope-induced fusion only the presence of GP120, but not virus replication or synthesis or viral proteins is required (Nature 322, 470-474 (1986)). HIV infection can be inhibited by CD4 monoclonal antibodies (Nature 312, 763-767 (1986)) and, as has been demonstrated, by actual binding of GP120 (envelope) to CD4 (Science 231, 382-385 (1986)).

Since the giant cells have a very short life time after they have been formed, cell fusion may be a major mechanism by which CD4-cell depletion, observed in AIDS and AIDS-related complex patients, is brought about. A direct lethal effect of HIV infection is somewhat unlikely since only one in 10'000 mononuclear cells are infected in these patients (Proc. Natl. Acad. Sci.USA 83, 772-776 (1986)). The viral envelope glycoprotein (GP120) is a heavily glycosylated protein and displays a high degree of variability in amino-acid sequence between viral isolates (Science 226, 1165-1171 (1984); Nature 312, 760-763 (1984); Science 229, 759-762 (1985); Science 228, 1091-1094 (1985)). Glycosylation seems to play an important role in HIV infectivity (J. Exp. Med. 164, 2101-2106 (1986); Science 234, 1392-1395 (1986)).

The effect of the inhibitor of oligosaccharide processing, 1-deoxynojirimycin, on HIV-induced cytopathic effects has now been studied in vitro. The following experiment shows that this compound is capable of preventing the cytopathic effect induced by HIV-infected B-cells. An Epstein-Barr virus-transformed B-cell line, infected with HTLV-IIIB, was cultured for at least 72 hours (in order to reduce the level of non-modified GP120 due to normal glycoprotein turnover) in the presence of 1-deoxynojirimycin. No clear toxic effects could be observed. The treated cells were then mixed with non-infected $CD4^{+}$H9 cells (Science 224, 497-500 (1984)). B-cells that were not treated with 1-deoxynojirimycin induced a strong cytopathic effect within 3 hours that could be inhibited by OKT4A monoclonal antibody, neutralizing human sera and concanavalin A. Infected B-cells that were treated with 3mM 1-deoxynojirimycin did not induce any cytopathic effect.

1-Deoxynojirimycin and the pharmaceutically acceptable acid addition salts thereof can be used, as mentioned earlier, for the treatment and/or prevention of AIDS and AIDS-related diseases caused by HIV. These compounds may be used as individual compounds or in combination with other therapeutically valuable substances which favourably influence the course of the disease. They may particularly be used in combination with agents that specifically interfere with the replication and/or expression of the viral genome of HIV or parts thereof such as 2',3'-dideoxycytidine (DDC) and 3'-azido-3'-deoxythymidine (AZT). The latter compounds may be administered previously, simultaneously or subsequently. In the case of combinations, 1-deoxynojirimycin and the pharmaceutically acceptable acid addition salts thereof may be used in the form of a comediacation, e.g. as part of a medicament pack, or as fixed combinations.

1-Deoxynojirimycin is preferably used in form of the free base.

1-Deoxynojirimycin and the pharmaceutically acceptable acid addition salts thereof are known compounds (German Offenlegungsschrift 27 26 898; Chem. Pharm. Bull. 34, 2642-2645 (1986)).

Objects of the present invention are: The use of 1-deoxynojirimycin and pharmaceutically acceptable acid addition salts thereof, optionally, in combination with other therapeutically valuable substances for the treatment and/or prevention of AIDS

and AIDS related diseases caused by HIV and for the manufacture of pharmaceutical compositions for the aforementioned purpose; pharmaceutical compositions comprising 1-deoxynojirimycin or a pharmaceutically acceptable acid addition salt thereof and an agent that specifically interferes with the replication and/or expression of the viral genome of HIV or parts thereof; as well as a method of treating and/or preventing AIDS and AIDS related diseases caused by HIV with 1-deoxynojirimycin and pharmaceutically acceptable acid addition salts thereof, optionally, in combination with other therapeutically valuable substances.

1-Deoxynojirimycin and the pharmaceutically acceptable acid addition salts thereof may be used in form of pharmaceutical compositions suitable for enteral or parenteral administration. The compounds in question can be administered, for example, perorally, e.g. in the form of tablets, coated tablets, dragees, hard and soft gelatine capsules, solutions, emulsions or suspensions, rectally, e.g. in the form of suppositories, or parenterally, e.g. in the form of injection solutions.

The manufacture of the pharmaceutical compositions can be effected in a manner which is familiar to any person skilled in the art by bringing the compounds in question, optionally in combination with the other therapeutically valuable substances, into a galenical administration form together with suitable, non-toxic, inert, therapeutically compatible solid or liquid carrier materials and, if desired, the usual pharmaceutical adjuvants.

As carrier materials there are suitable not only inorganic carrier materials, but also organic carrier materials. Thus, for tablets, coated tablets, dragees and hard gelatine capsules there can be used as carrier materials, for example, lactose, maize strach or derivatives thereof, talc, stearic acid or its salts. Suitable carriers for soft gelatine capsules are, for example, vegetable oils, waxes, fats and semi-solid and liquid polyols (depending on the nature of the active substance no carriers are, however, required in the case of soft gelatine capsules). Suitable carrier materials for the manufacture of solutions and syrups are, for example, water, polyols, saccharose, invert sugar and glucose. Suitable carrier materials for injection solutions are, for example, water, alcohols, polyols, glycerine and vegetable oils. Suitable carrier materials for suppositories are, for example natural or hardened oils, waxes, fats and semi-liquid or liquid polyols.

As pharmaceutical adjuvants there come into consideration the usual preserving agents, solubilizers, stabilizing agents, wetting agents, emulsifying agents, flavour-improving agents such as sweetening agents and flavouring agents, colouring agents, salts for varying the osmotic pressure, buffers, coating agents and antioxidants.

The dosage of the described substances can vary within wide limits depending on the severity of the illness to be treated, the age and the individual condition of the patient and on the mode of administration and will, of course, be fitted to the individual requirements in each particular case. In the treatment or prevention of AIDS and/or AIDS related diseases a daily dosage of 100 mg to about 2000 mg, especially about 200 mg to about 1000 mg, comes into consideration. Depending on the dosage it is convenient to administer the daily dosage in several dosage units.

The preferred pharmaceutical compositions in accordance with the invention are those which can be administered orally. They conveniently contain about 100 mg to about 2000 mg, preferably about 200 mg to about 1000 mg, of 1-deoxynojirimycin or of a pharmaceutically acceptable acid addition salt thereof.

## Claims

1. The use of 1-deoxynojirimycin of the formula

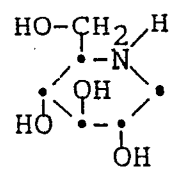

I

or of a pharmaceutically acceptable acid addition salt thereof, optionally, in combination with an agent that specifically interferes with the replication and/or expression of the viral genome of HIV or parts thereof and/or an other therapeutically valuable substance in the manufacture of pharmaceutical compositions for the treatment and/or prevention of AIDS and AIDS related diseases caused by HIV.

2. The use of 1-deoxynojirimycin in form of the free base, optionally, in combination with an agent that specifically interferes with the replication and/or expression of the viral genome of HIV or parts thereof and/or an other therapeutically valuble usbstance in the manufacture of pharmaceutical compositions for the treatment and/or prevention of AIDS and AIDS related diseases caused by HIV.

3. The use of 1-deoxynojirimycin in form of free base in the manufacture of pharmaceutical compositions for the treatment and/or prevention of AIDS and AIDS related diseases caused by HIV.

4. A pharmaceutical composition comprising 1-deoxynojirimycin of the formula

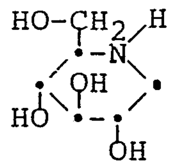

I

or a pharmaceutically acceptable acid addition salt thereof, an agent that specifically interferes with the replication and/or expression of the viral genome of HIV or parts thereof and a therapeutically inert carrier material.

5. A pharmaceutical composition according to claim 4, comprising 1-deoxynojirimycin in form of the free base.

6. A pharmaceutical composition according to claim 4 or 5, wherein said agent is 3′-azido-3′-deoxythymidine or 2′,3′--dideoxycytidine.

7. A method of treating and/or preventing AIDS and AIDS related diseases caused by HIV in a human being which comprises administering to said patient an effective amount of 1-deoxynojirimycin of the formula

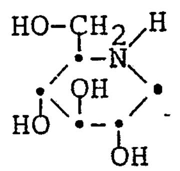

I

or a pharmaceutically acceptable acid addition salt thereof together with a therapeutically inert carrier material.

8. A method in accordance with claim 7, wherein 1-deoxynojirimycin is usedin form of the free base.

9. A method in accordance with claim 7 or 8, wherein an effective amount of an agent that specifically interferes with the replication and/or expression of the viral genome of HIV or parts thereof is administered previously, simultaneously or subsequently.

10. A method in accordance with claim 9, wherein said agent is 3′-azido-3′-deoxythymidine or 2′,3′-dideoxycytidine.

11. The use of 1-deoxynojirimycin of the formula

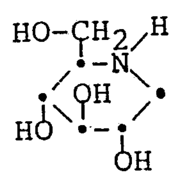

I

or of a pharmaceutically acceptable acid addition salt thereof, optionally, in combination with an agent that specifically interferes with the replication and/or expression of the viral genome of HIV or parts thereof or an other therapeutically valuable substance for the treatment and/or prevention of AIDS and AIDS related diseases caused by HIV.

12. The use of 1-deoxynojirimycin in form of the free base, optionally, in combination with an agent that specifically interferes with the replication and/or expression of the viral genome of HIV or parts thereof or an other therapeutically valuable substance for the treatment and/or prevention of AIDS and AIDS related diseases caused by HIV.

13. The use of 1-deoxynojirimycin in form of the free base for the treatment and/or prevention of AIDS and AIDS related diseases caused by HIV.

14. The use of 1-deoxynojirimycin in form of the free base, optionally, in combination with 3′-azido-3′-deoxythymidine or 2′,3′-dideoxycytidine for the treatment and/or prevention of AIDS and AIDS related diseases caused by HIV.

15. The invention as hereinbefore described.

European Patent Office

**PARTIAL EUROPEAN SEARCH REPORT**
which under Rule 45 of the European Patent Convention shall be considered, for the purposes of subsequent proceedings, as the European search report

Application number
EP 87 10 3995

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | VIROLOGY, vol. 136, no. 1, July 15, 1984, pages 196-210; Academic Press Inc., US<br>A. PINTER et al.: "Studies with inhibitors of oligosaccharide processing indicate a functional role for complex sugars in the transport and proteolysis of friend mink cell focus-inducing murine leukemia virus envelope proteins."<br>* Whole article *<br>--- | 1-6 | A 61 K 31/445 |
| Y | EMBO JOURNAL, vol. 4, no. 1, January 1985, pages 105-112; IRL Press Ltd, Oxford, GB<br>J.A. SCHMIDT et al.: "Effects of inhibitors of glycoprotein processing on the synthesis and biological activity of the erb B oncogene"<br>* Whole article *<br>---<br>./. | 1-6 | TECHNICAL FIELDS SEARCHED (Int. Cl.4)<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6
Claims searched incompletely:
Claims not searched: 7-14 and 15
Reason for the limitation of the search:

For claims 7-14:
Method for treatment of the human or animal body by surgery or therapy (see art. 52(4) of the European Patent Convention).

For claim 15:
Rule 29(6) of the European Patent Convention.

| Place of search | Date of completion of the search | Examiner |
|---|---|---|
| The Hague | 06-10-1987 | THEUNS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03 82

European Patent Office

# PARTIAL EUROPEAN SEARCH REPORT

Application number EP 87 10 3995



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 196 185 (WELLCOME)<br>* Whole document * | 1-6 | |
| Y | PROC. NATL. ACAD. SCI. USA, vol. 83, March 1986, pages 1911-1915; US H. MITSUYA et al.: "Inhibition of the in vitro infectivity and cytopathic effect of human T-lymphotrophic virus type III/lymphadenopathy-associated virus (HTLV-III/LAV) by 2',3'-dideoxynucleosides."<br>* Whole article * | 1-6 | |
| E | WO-A-87 03 903 (F. HUTCHINSON CANCER RES. CENTER)<br>* Whole document * | 1-6 | |

TECHNICAL FIELDS SEARCHED (Int. Cl.4)